# EUROPÄISCHE PATENTANMELDUNG

(11) **EP 4 726 358 A1**
(43) Veröffentlichungstag der Anmeldung: **15.04.2026**
(21) Anmeldenummer: 25206818.4
(22) Anmeldetag: 06.10.2025
(51) Int. Cl.: G01N 11/00, A61B 5/00

(54) **MOBILE RHEOMETERVORRICHTUNG, SYSTEM UND VERFAHREN ZUR UNTERSUCHUNG VON RHEOLOGISCHEN EIGENSCHAFTEN VON MENSCHLICHEN, TIERISCHEN ODER PFLANZLICHEN BESTANDTEILEN**

(30) Priorität: 09.10.2024 DE 102024129204
(71) Anmelder: Netzsch-Gerätebau GmbH, 95100 Selb (DE)
(72) Erfinder: Küspert, Sabrina, 95100 Selb (DE); Rummel, Florian, 95100 Selb (DE); Szántó, Levente, 95028 Hof (DE); Taubmann, Rebekka, 95100 Selb (DE)

(57) **Zusammenfassung**

Die vorliegende Erfindung betrifft eine mobile Rheometervorrichtung (1) zur Untersuchung von rheologischen Eigenschaften von menschlichen, tierischen oder pflanzlichen Bestandteilen. Solch eine mobile Rheometervorrichtung (1) umfasst dabei eine rheologische Messeinrichtung (4) und eine damit gekoppelte computerbasierte Funktionseinheit (2) mit Anwenderprogramm (3). Die mobile Rheometervorrichtung (1) umfasst zudem eine Transporteinheit (6), in welcher die rheologische Messeinrichtung (4) und die damit gekoppelte computerbasierte Funktionseinheit (2) angeordnet sind, wobei die Transporteinheit (6) ausgelegt ist, zumindest temporär in einem inneren Bestandteil eines menschlichen, tierischen oder pflanzlichen Körpers platziert zu werden, sodass die rheologische Messeinrichtung (4) mit dem menschlichen, tierischen oder pflanzlichen Körper unter Kontaktaufnahme interagieren kann.

## Beschreibung

### TECHNISCHES GEBIET DER ERFINDUNG

Die vorliegende Erfindung betrifft eine mobile Rheometervorrichtung, ein System und ein Verfahren zur Untersuchung von rheologischen Eigenschaften von menschlichen, tierischen oder pflanzlichen Bestandteilen.

### HINTERGRUND DER ERFINDUNG

Um weitergehende Aussagen über menschliche, tierische oder pflanzliche Bestandteile, beispielsweise in Form von einzelnen Proben, treffen zu können, werden diese Bestandteile oder nur Proben davon häufig nach der Entnahme vom Organismus aufwendig im Labor analysiert. Alternativ ist auch bekannt, eine im Zusammenhang mit dem zu untersuchendem Gewebe oder Organismus stehende Flüssigkeit zu untersuchen.

So werden beispielsweise Proben von einem Menschen während einer Biopsie oder einer Operation durch medizinisches Fachpersonal entnommen und anschließend zur Pathologie gebracht. In der Pathologie erfolgt anschließend häufig eine histologische Untersuchung, typischerweise mit Färbung von Gewebe und nachfolgender pathologischer Diagnostik. Auch können Gewebeproben mittels Gewebeschnitte aufbereitet werden, um diese einzelnen Schnitte jeweils anschließend weiter zu analysieren.

Anstatt einer histologischen Untersuchung mit Einfärbung und Einsatz von mikroskopischen Geräten können auch rheologische Techniken verwendet werden, um etwa eine medizinische Diagnostik zu betreiben. Diese Techniken können beispielsweise Rotationsrheometer oder Kapillarrheometer umfassen. Auch können Kugelfallviskosimeter oder mikrofluidische Vorrichtungen eingesetzt werden. Pathologisches Gewebe unterscheidet sich vom physiologischen Zustand beispielsweise durch seine viskosen, viskoelastischen oder elastischen Eigenschaften.

Beispielsweise lassen sich auf diese Weise Stenosen oder vergleichbare Leiden näher untersuchen. Körperflüssigkeiten, wie etwa Speichel, Blut et cetera, unterscheiden sich ebenfalls in ihren viskosen, viskoelastischen oder elastischen Eigenschaften, wenn sich eine Veränderung einstellt. Dieser Umstand ist zum Beispiel im Zusammenhang mit der Sichelzellenanämie bekannt. Insofern lassen sich Kenntnisse über rheologische Eigenschaften von physiologischen oder pathologischen Eigenschaften zur Diagnostik verwenden.

Dabei können Messungen von einer oder mehrerer rheologischen Größen, wie beispielsweise (Scher-)Viskosität, Dehnviskosität, Schubspannung, viskoelastische Eigenschaften, Speichermodul, Verlustmodul, gemessen werden. Solche Messungen können absolute oder relative Messwerte umfassen. Typischerweise wird eine jeweilige Probe hierzu in Oszillation, Schwingung versetzt. Alternativ kann auch eine Rotation oder eine Bewegung in Form einer Strömung vorgesehen sein. Auch körpergegebene Umstände von Proben können bereits Formen von Oszillation, Schwingung, Rotation oder Strömungen bedingen, sodass dieser Umstand für rheologische Messverfahren genutzt werden kann.

Die skizzierten Vorgehensweisen mit den jeweiligen einzusetzenden Techniken gehen mit einem gewissen Zeitaufwand einher. Zudem wird für den Transport der Proben eine bestimmte Logistikinfrastruktur benötigt, um die häufig sensiblen Proben während dieser Zeit nicht zu beschädigen. In diesem Zusammenhang sind trotz aller Vorsichtsmaßnahmen Veränderungen der Proben wahrscheinlich und können nicht gänzlich ausgeschlossen werden. Generell ändert sich biologisches Probenmaterial zeitlich schnell, sodass nachfolgende Untersuchungen beziehungsweise die Analytik erschwert wird.

Für die oben erwähnten Fälle der Biopsie und der Entnahme während einer Operation ist die nötige medizinische Infrastruktur häufig nur bei Maximalversorgern gegeben. Eine Versorgung und Diagnostik in entlegenen Gegenden, wie beispielsweise im Hochgebirge oder auf hoher See, kann sich somit als schwierig erweisen, da die nötige medizinische Infrastruktur entweder nur schwer bereitstellbar oder schlicht nicht gegeben ist.

Um zudem Messungen von einer oder mehrerer rheologischen Größen kontinuierlich erheben zu können, sind bereits bekannte stationäre Gerätschaften oft zu unhandlich oder schlicht zu groß, als dass sie von einem Patienten während eines längeren Zeitraums bei verschiedensten Aktivitäten komfortabel genutzt werden könnten. Ausschließlich äußerlich anwendbare Geräte können während der Nutzungsphase am menschlichen, tierischen oder pflanzlichen Organismus zudem verrutschen und so unter Umständen die zu erhebenden Messergebnisse empfindlich verfälschen. Damit einher gehen dann oft kostspielige durchzuführende Wiederholungsmessungen. Gerade bei einer dringend bereitzustellenden Diagnose ist der Faktor Zeit dabei kritisch zu betrachten.

### ZUSAMMENFASSUNG DER ERFINDUNG

Vor diesem Hintergrund liegt der vorliegenden Erfindung die Aufgabe zugrunde eine mobile Rheometervorrichtung zur Untersuchung von rheologischen Eigenschaften von menschlichen, tierischen oder pflanzlichen Bestandteilen sowie ein System und ein Verfahren bereitzustellen, welche zumindest teilweise die zuvor genannten Nachteile überkommen.

Diese Aufgabe wird gelöst durch ein mobile Rheometervorrichtung mit den Merkmalen des Patentanspruchs 1 sowie durch eine System mit den Merkmalen des Patentanspruchs 26 und ein Verfahren mit den Merkmalen des Patentanspruchs 27.

Dementsprechend ist eine mobile Rheometervorrichtung zur Untersuchung von rheologischen Eigenschaften von menschlichen, tierischen oder pflanzlichen Bestandteilen vorgesehen. Solch eine mobile Rheometervorrichtung umfasst dabei eine rheologische Messeinrichtung und eine damit gekoppelte computerbasierte Funktionseinheit mit Anwenderprogramm Die mobile Rheometervorrichtung umfasst dabei eine Transporteinheit in welcher die rheologische Messeinrichtung und die damit gekoppelte computerbasierte Funktionseinheit angeordnet sind. Diese Transporteinheit ist in diesem Zusammenhang ausgelegt, zumindest temporär in einem inneren Bestandteil eines menschlichen, tierischen oder pflanzlichen Körpers platziert zu werden, sodass die rheologische Messeinrichtung mit dem menschlichen, tierischen oder pflanzlichen Körper unter Kontaktaufnahme interagieren kann.

Des Weiteren ist ein System zur Untersuchung von rheologischen Eigenschaften von menschlichen, tierischen oder pflanzlichen Bestandteilen vorgesehen, welches zumindest eine erfindungsgemäße mobile Rheometervorrichtung und eine mit der zumindest einen erfindungsgemäßen mobilen Rheometervorrichtung kontaktlos koppelbaren externen Steuereinrichtung mit Applikationsprogramm umfasst. Ferner umfasst das System in dieser Basisvariante wenigstens eine mit der Steuereinrichtung koppelbare Datenbank für die medizinische Diagnostik von Bestandteilen eines menschlichen, tierischen oder pflanzlichen Körpers, wobei die Datenbank auf rheologischen Parametern basiert.

Zudem ist ein Verfahren zur Untersuchung von rheologischen Eigenschaften von menschlichen, tierischen oder pflanzlichen Bestandteilen vorgesehen, welches die folgenden Verfahrensschritte umfasst: Bereitstellen und Aktivieren einer erfindungsgemäßen mobilen Rheometervorrichtung, Platzieren der mobilen Rheometervorrichtung in oder an einem Bestandteil von einem menschlichen, tierischen oder pflanzlichen Körper, Auslösen von wenigstens einer Funktion der rheologischen Messeinrichtung von der mobilen Rheometervorrichtung zur Interaktion mit wenigstens einem Teilbereich des Bestandteils des menschlichen, tierischen oder pflanzlichen Körpers, sodass wenigstens ein rheologischer Parameter mittels der rheologischen Messeinrichtung gemessen und bereitgestellt wird.

Auch wird eine weitere Verwendung der erfindungsgemäßen mobilen Rheometervorrichtung offenbart. Es ist vorgesehen, die erfindungsgemäße mobile Rheometervorrichtung für Durchführungen von therapeutischen oder kurierenden Maßnahmen in einem menschlichen, tierischen oder pflanzlichen Organismus zu verwenden.

Eine der Erfindung zugrunde liegende Idee besteht darin, eine mobile Rheometervorrichtung bereitzustellen, mit welcher Messungen von einer oder mehrerer rheologischer Größen, wie beispielsweise Viskositäten, Scherviskositäten, Dehnviskositäten, Schubspannungen, viskoelastische Eigenschaften, Speichermodul, Verlustmodul, in oder an einem Bestandteil von einem menschlichen, tierischen oder pflanzlichen Körper durchgeführt werden können und die Messergebnisse für eine weitergehende Diagnostik bereitgestellt werden können. Die durchgeführten Messungen der dabei eingesetzten rheologischen Messeinrichtung im Zuge einer Interaktion mit dem jeweiligen Bestandteil des menschlichen, tierischen oder pflanzlichen Körpers können dabei absolute und relative Messwerte umfassen.

Die vorgesehene mobile Rheometervorrichtung ist dabei mit Vorteil so beschaffen, sodass sie innerhalb eines Bestandteils des menschlichen, tierischen oder pflanzlichen Körpers eingesetzt werden kann. Eine für diese Zwecke ausgelegte Transporteinheit ermöglicht diese Form des Einsatzes innerhalb des zu untersuchenden menschlichen, tierischen oder pflanzlichen Körpers. Als zu untersuchende Bestandteile können in diesem Zusammenhang jegliche Subeinheiten innerhalb des menschlichen, tierischen oder pflanzlichen Körpers verstanden werden.

Beispielsweise kann es sich bezogen auf einen Menschen um eine Organeinheit des Körpers oder allgemein um eine komplexere Struktur innerhalb des menschlichen Körpers, beispielsweise eine besondere Knochenstruktur und so weiter, handeln.

Die vorgesehene erfindungsgemäße mobile Rheometervorrichtung kann auch an dem zu untersuchenden Bestandteil des menschlichen, tierischen oder pflanzlichen Körpers beziehungsweise Organismus eingesetzt werden. Auch außerhalb des jeweiligen Körpers ist ein Einsatz vorstellbar, beispielsweise um vor dem eigentlichen Einsatz im Inneren des Körpers mögliche Kalibrierungsschritte oder dergleichen durchzuführen.

Der Vorteil der vorgesehenen mobilen Rheometervorrichtung liegt unter anderem darin begründet, dass auf überraschend einfache Weise sichergestellt werden kann, dass eine oder mehrere rheologische Größen kontinuierlich erhoben werden können, wobei aufgrund einer handlichen Vorgehensweise eine komfortable Bedienung resultiert.

Dadurch, dass zum Zwecke der Messung dieser Größen eine unmittelbare Kontaktierung des jeweiligen zu untersuchenden Bestandteils vorgesehen ist, kann zudem eine sichere und folgerichtige Messweise gewährleistet werden. Auch können zudem längere Messzeiträume mittels der erfindungsgemäßen mobilen Rheometervorrichtung vorgesehen sein, da der Einsatz unmittelbar am zu untersuchenden Objekt ohne weitere Einschränkungen für den zu untersuchenden Organismus einhergeht. Beispielsweise kann in einem Menschen die mobile Rheometervorrichtung so platziert werden, sodass der Patient dadurch in keiner nennenswerten Art und Weise in seinen Lebensaktivitäten eingeschränkt wird.

Die vorgestellte mobile Rheometervorrichtung begünstigt zudem eine schnelle Vorgehensweise bei der Ermittlung von Messergebnissen, da keine weiteren Transporte von zuvor entnommenen Proben zur eigentlichen Messeinrichtung nötig sind. Auch entfällt mit der vorgesehenen mobilen Rheometervorrichtung eine aufwendige gesonderte Aufbereitung des zu untersuchenden Bestandteils.

Die für die rheologischen Messungen nötigen Interaktionen sind schnell und mit einem überschaubaren zeitlichem Aufwand in nahezu Echtzeit durchführbar, sodass im Wesentlichen unmittelbar anschließend sofort eine jeweilige Messung oder eine Messreiche durchgeführt werden kann.

Eine Beschädigung von zu bemessenden Teilbereichen kann auch weitestgehend ausgeschlossen werden, sodass auf vorteilhafte Weise eine besonders sichere Messerhebungsmethode mittels der erfindungsgemäßen Vorrichtung bereitgestellt werden kann.

Die erfindungsgemäße mobile Rheometervorrichtung ist zudem mit Vorteil auch im Zuge einer Versorgung und Diagnostik in entlegeneren Gegenden einsetzbar, wie beispielsweise im Hochgebirge oder auf hoher See. Die mittels größeren Gerätschaften einhergehende schwierige Logistik und Einrichtung von einer aufwendigen Infrastruktur entfällt, sodass eine flexible und leicht zu bedienende erfindungsgemäße mobile Rheometervorrichtung hier weitere Vorteile bietet.

Gemäß einer weiteren Ausführungsvariante der mobilen Rheometervorrichtung ist vorgesehen, dass die Transporteinheit Befestigungsmittel umfasst, welche ausgelegt sind, eine temporäre oder dauerhafte Befestigung der mobilen Rheometervorrichtung in dem inneren Bestandteil des menschlichen, tierischen oder pflanzlichen Körpers zu ermöglichen.

Auf diese Weise kann sichergestellt werden, dass auch längere Messungen im Sinne etwa von wiederkehrenden Messungen in bestimmten Zeitintervallen oder dergleichen jeweils im Wesentlichen an einem gleichen Teilbereich des zu untersuchenden Bestandteils des Körpers komfortabel und mit Vorteil durchgeführt werden können.

Nach einer erstmaligen Platzierung kann die mobile Rheometervorrichtung so im Wesentlichen an Ort und Stelle verbleiben, sodass die gewünschten Messungen komfortabel und folgerichtig durchgeführt werden können. Auch kann somit eine benutzerdefinierte Platzierung ermöglicht werden, welche sich beispielsweise als besonders schonend für den zu untersuchenden Organismus darstellt.

Gemäß einer Weiterbildung der mobilen Rheometervorrichtung ist vorgesehen, dass die Befestigungsmittel ausgewählt sind aus: anorganische Befestigungsmittel, organische Befestigungsmittel, anorganische Befestigungsmittel, welche ausgelegt sind, eine mechanische Verbindung zu ermöglichen, organische Befestigungsmittel, welche ausgelegt sind, eine mechanische Verbindung zu ermöglichen, anorganische Befestigungsmittel, welche ausgelegt sind, eine lösbare mechanische Verbindung zu ermöglichen, organische Befestigungsmittel, welche ausgelegt sind, eine lösbare mechanische Verbindung zu ermöglichen, anorganische Befestigungsmittel, welche ausgelegt sind, eine formschlüssige Verbindung mit zumindest einem Teilbereich des inneren Bestandteils zu ermöglichen, organische Befestigungsmittel, welche ausgelegt sind, eine formschlüssige Verbindung mit zumindest einem Teilbereich des inneren Bestandteils zu ermöglichen, anorganische Befestigungsmittel, welche ausgelegt sind, eine kraftschlüssige Verbindung mit zumindest einem Teilbereich des inneren Bestandteils zu ermöglichen, organische Befestigungsmittel, welche ausgelegt sind, eine kraftschlüssige Verbindung mit zumindest einem Teilbereich des inneren Bestandteils zu ermöglichen, anorganische Befestigungsmittel, welche ausgelegt sind, eine stoffschlüssige Verbindung mit zumindest einem Teilbereich des inneren Bestandteils zu ermöglichen, organische Befestigungsmittel, welche ausgelegt sind, eine stoffschlüssige Verbindung mit zumindest einem Teilbereich des inneren Bestandteils zu ermöglichen.

Je nach Einsatzgebiet und Einsatzort kann somit ein hierfür geeignetes Befestigungsmittel vorgesehen sein, welches für die jeweiligen nötigen Zwecke sich als besonders vorteilhaft darstellt. So kann beispielsweise in einem menschlichen Körper ein organisches Befestigungsmittel sich als besonders schonend und für den Organismus besonders gut verträglich darstellen.

Gemäß einer Weiterbildung der mobilen Rheometervorrichtung ist vorgesehen, dass die Befestigungsmittel zumindest teilweise auf einer Oberfläche der Transporteinheit angeordnet sind. Auf diese Weise kann nach der Platzierung der Vorrichtung unter Kontaktaufnahme mit dem jeweils zu untersuchenden Bestandteil des jeweiligen Körpers beziehungsweise Organismus eine unmittelbare Befestigung erfolgen, da aufgrund der oben genannten speziellen Anordnung eine unmittelbare Verbindung vorgesehen werden kann.

Gemäß einer weiteren Ausführungsvariante der mobilen Rheometervorrichtung ist vorgesehen, dass die Befestigungsmittel organische Befestigungsmittel umfassen, welche ausgelegt sind, mittels wenigstens einem benutzerdefiniert eingebrachten Einfluss manipulierbar zu sein, sodass die organischen Befestigungsmittel an eine molekulare Struktur des inneren Bestandteils eines menschlichen, tierischen oder pflanzlichen Körpers anpassbar sind.

Eine besonders gezielte und somit besonders stabile Verbindung kann somit erzielt werden, sodass die erfindungsgemäße Vorrichtung mit Vorteil benutzerfreundlich und zuverlässig eingesetzt werden kann. Insbesondere auch im Sinne der bereits zuvor genannten Vorteile kann mittels eines derartig speziell angepassten Befestigungsmittel eine schonende und somit gut verträgliche technische Lösung für längere Messzeiträume bereitgestellt werden.

Gemäß einer weiteren Ausführungsvariante der mobilen Rheometervorrichtung ist vorgesehen, dass der benutzerdefiniert eingebrachte Einfluss ausgewählt ist aus: thermischer Einfluss, elektromechanischer Einfluss, mechanischer Einfluss, chemischer Einfluss, chemische, biologische oder physikalische Übertragung von Energie.

Je nach Einsatzzweck kann somit ein jeweils optimales Befestigungsmittel, welches mittels der jeweiligen Technik vorbereitet werden kann, bereitgestellt werden. Der jeweils eingebrachte Einfluss kann etwa kurz vor der eigentlichen Platzierung der Vorrichtung vorgenommen werden.

Gemäß einer Weiterbildung der mobilen Rheometervorrichtung ist vorgesehen, dass die mobile Rheometervorrichtung zumindest eine Antriebseinheit umfasst, sodass die mobile Rheometervorrichtung innerhalb des inneren Bestandteils des menschlichen, tierischen oder pflanzlichen Körpers bewegbar ausgestaltet ist.

Auf diese Weise kann zusätzlich zu einer ersten Platzierung die mobile Rheometervorrichtung besonders ortsgenau zu einer gewünschten Stelle in Bezug auf den zu untersuchenden Bestandteil navigiert werden. Im Sinne einer Feinjustierung kann somit ein besonders vorteilhafter Messort ausgewählt werden, sodass die nachfolgenden Messungen die gewünschte Diagnostik oder dergleichen bestmöglich gewährleisten.

Gemäß einer weiteren Ausführung der mobilen Rheometervorrichtung ist vorgesehen, dass die zumindest eine Antriebseinheit ausgelegt ist, eine Bewegung der mobilen Rheometervorrichtung in einer Flüssigkeit, in welcher sich die mobile Rheometervorrichtung befindet, zu bewirken oder wobei die zumindest eine Antriebseinheit ausgelegt ist, eine Bewegung der mobilen Rheometervorrichtung auf einem im Wesentlichen festen Untergrund, auf welchem sich die mobile Rheometervorrichtung befindet, zu bewirken oder wobei die zumindest eine Antriebseinheit ausgelegt ist, eine Bewegung der mobilen Rheometervorrichtung in einer Flüssigkeit, in welcher sich die mobile Rheometervorrichtung befindet, und eine Bewegung der mobilen Rheometervorrichtung auf einem im Wesentlichen festen Untergrund, auf welchem sich die mobile Rheometervorrichtung befindet, zu bewirken.

Für die jeweiligen Umgebungsbindungen im inneren Bestandteil des menschlichen, tierischen oder pflanzlichen Körpers kann somit eine gewählte Art und Weise der Antriebseinheit vorgesehen werden, sodass eine optimale Platzierung der entsprechend ausgebildeten mobilen Rheometervorrichtung besonders ortsgenau an einer gewünschten Stelle in Bezug auf den zu untersuchenden Bestandteil erreicht werden kann. Eine vorteilhafte Navigation kann somit noch gezielter im Sinne eines Erreichens eines besonders vorteilhaften Messorts gewährleistet werden.

Gemäß einer weiteren Ausführungsvariante ist vorgesehen, dass die zumindest eine Antriebseinheit zumindest eine Taptic-Motoreinheit umfasst.

Die mittels der Taptic-Motoreinheit bewirkten Vibrationen können kleinteilige Bewegungsimpulse der mobilen Rheometervorrichtung begünstigen, welche bereits für eine finale Platzierung ausreichend sein können. Insbesondere ist diese Vorgehensweise dann von Vorteil, wenn nur kleinere Verlagerungsbewegungen der mobilen Rheometervorrichtung gewünscht sind, beispielsweise um eine bereits erreichte Position zu halten. Dies kann zudem insbesondere dann von Vorteil sein, wenn äußere Einflüsse von dem inneren Bestandteil des menschlichen, tierischen oder pflanzlichen Körpers immer wieder zu kleineren Verschiebungen der mobilen Rheometervorrichtung weg vom optimalen Messort bewirken.

Gemäß einer alternativen Ausführung der mobilen Rheometervorrichtung ist vorgesehen, dass die zumindest eine Antriebseinheit ausgelegt ist, mittels der computerbasierten Funktionseinheit gesteuert zu werden.

Neben der Möglichkeit, jeweilige Navigationskorrekturen gezielter vornehmen zu können, besteht zusätzlich die Möglichkeit auch entferntere weitere Messorte gezielt anzusteuern. Mit anderen Worten bietet eine steuerbare Antriebseinheit weitere Vorteile, um eine noch bessere Diagnostik letztendlich zu erhalten, in dem möglichst viele Messwerte an unterschiedlichen Positionen erhoben werden können. Eine erstmalige Platzierung, welche unter Umständen mit einer einfachen Antriebseinheit ohne eine gesonderte Steuerung lediglich in kleinerem Umfang nachjustiert werden kann, kann mittels dieser Ausführungsvariante benutzerdefiniert auch in größeren Untersuchungsbereichen neu ausgerichtet werden, sodass auch an weiter beabstandeten Messorten jeweils gemessen werden kann.

Gemäß eines weiteren Ausführungsbeispiels ist vorgesehen, dass die zumindest eine Antriebseinheit zumindest anteilig aus organischem Material besteht.

Der Vorteil liegt hier insbesondere darin, dass nicht nur eine verträgliche Implementierung in dem inneren Bestandteil des menschlichen, tierischen oder pflanzlichen Körpers somit möglich ist, sondern auch bei gegebenenfalls längeren Manövern sich diese Verträglichkeit auch nicht verschlechtert.

Eine zumindest anteilig aus einem organischen Material bestehende Antriebseinheit zeichnet sich mitunter dadurch aus, nicht unmittelbar als Fremdkörper wahrgenommen zu werden, sodass die Wahrscheinlichkeit von unmittelbaren negativen Reaktionen des menschlichen, tierischen oder pflanzlichen Körpers als gering angesehen werden kann.

Gemäß einer weiteren Ausführungsvariante ist vorgesehen, dass die zumindest eine Antriebseinheit zumindest eine bewegliche Außenmembranstruktur umfasst, welche außerhalb an zumindest einem Teilbereich der Transporteinheit angeordnet ist, sodass mittels pulsierender Bewegungen der jeweiligen beweglichen Außenmembranstruktur eine definierte Bewegung der mobilen Rheometervorrichtung bewirkbar ist.

Auf diese Weise kann eine besonders kompakte mobile Rheometervorrichtung bereitgestellt werden, welche zudem keine mitunter als störend empfundenen abstehenden Bestandteile umfasst. Vielmehr kann sich solch eine bewegliche Außenmembranstruktur einer bereits vorhandenen Hauptform der mobilen Rheometervorrichtung anpassen, sodass die zuvor genannten kompakten Außenmaße gewährleistet werden können.

Gemäß eines weiteren Ausführungsbeispiels ist vorgesehen, dass die zumindest eine bewegliche Außenmembranstruktur mittels der für diese Zwecke mit der jeweiligen Außenmembranstruktur gekoppelten computerbasierten Funktionseinheit mit Anwenderprogramm bewegbar ist oder wobei die zumindest eine bewegliche Außenmembranstruktur mittels einer für diese Zwecke mit der jeweiligen Außenmembranstruktur kontaktlos gekoppelten externen Anregungseinheit mit Anwenderprogramm bewegbar ist.

Mittels einer steuerbaren beweglichen Außenmembranstruktur sind die zuvor genannten Vorteil in beiden Ausführungsvarianten, sowohl mittels der gekoppelten computerbasierten Funktionseinheit mit Anwenderprogramm als auch mittels der kontaktlos gekoppelten externen Anregungseinheit mit Anwenderprogramm, jeweils noch besser zu erreichen.

Eine Anregungseinheit mit Anwenderprogramm kann beispielsweise als ein jegliches mobiles Steuergerät, etwa ein Tablet oder ein Mobiltelefon jeweils mit einem passenden Anwenderprogramm, beispielsweise in Form einer Applikation oder dergleichen, ausgestattet, vorgesehen sein, sodass diese Funktion nicht zwingend in der mobilen Rheometervorrichtung vorgehalten sein muss, was wiederum zu einer weniger komplexen und somit anwenderfreundlichen Vorrichtung führt.

Gemäß einer weiteren Ausführungsvariante der mobilen Rheometervorrichtung ist vorgesehen, dass die zumindest eine Antriebseinheit ausgelegt ist, eine benutzerdefinierte Geschwindigkeit, insbesondere eine benutzerdefiniert gleichmäßige Beschleunigung oder eine benutzerdefiniert negative Beschleunigung oder eine benutzerdefiniert positive Beschleunigung zu bewirken.

Diese jeweiligen Funktionen können entweder voreingestellt oder mittels einer entsprechenden Steuerung vorgesehen werden. Die damit einhergehenden Effekte können jeweils für eine gezieltere Handhabung der mobilen Rheometervorrichtung genutzt werden. So kann etwa eine benutzerdefiniert positive Beschleunigung in Richtung eines Gewebebereiches eine besonders stabile Befestigung der mobilen Rheometervorrichtung an diesem Messort begünstigen.

Gemäß einer weiteren Ausführungsvariante der mobilen Rheometervorrichtung ist vorgesehen, dass die zumindest eine Antriebseinheit ausgelegt ist, eine jegliche Bewegung oder einen jeglichen Bewegungsablauf der mobilen Rheometervorrichtung in einem dreidimensionalen Koordinatensystem zu bewirken. Die zuvor genannten Vorteile sind somit noch besser erreichbar.

Gemäß eines weiteren Ausführungsbeispiels ist vorgesehen, dass die computerbasierte Funktionseinheit eine Speichereinheit zur Speicherung von rheologischen Messdaten umfasst.

Somit müssen die erhobenen Messdaten nicht sofort an weitere Geräte übermittelt werden, sondern können vielmehr zeitversetzt zu einem späteren Zeitpunkt aus der Speichereinheit abgerufen werden. Dies kann etwa dann der Fall sein, wenn die mobile Rheometervorrichtung aus dem jeweiligen Körper wieder entfernt wird und somit anschließend ein freier Zugang zu der Speichereinheit möglich ist.

Bei längeren Erhebungszeiträumen, etwa wenn ein Patient sich mit der implementierten mobilen Rheometervorrichtung frei bewegt, können somit die erhobenen Messdaten erst von der Speichereinheit gespeichert werden, ohne dass eine ständige Verbindung nach außen zu Empfangsgeräten nötig wäre.

Gemäß einer weiteren Ausführungsvariante der mobilen Rheometervorrichtung ist vorgesehen, dass die computerbasierte Funktionseinheit ein Sende- und Empfangsmodul umfasst, sodass die mobile Rheometervorrichtung ausgelegt ist, erhobene rheologische Messdaten an zumindest ein externes Gerät zu übermitteln und ferner ausgelegt ist, mittels zumindest eines externen Geräts bedient zu werden.

Dies bietet den Vorteil, dass (nach erfolgreicher Platzierung der mobilen Rheometervorrichtung) der zu bemessende Organismus, ob nun in menschlicher, tierischer oder pflanzlicher Form, relativ frei hinsichtlich seiner Verortung ist, da mittels des Sende- und Empfangsmoduls eine Verbindung zu externen Geräten auch über eine gewisse Distanz gewährleistet werden kann. Insofern kann eine weitestgehend lückenlose Dokumentation der erhobenen Messwerte auch in Echtzeit erreicht werden, was im Fall von kritischen Werten einen großen Zeitvorsprung bedeutet, sodass rasch etwa kurative Maßnahmen oder dergleichen veranlasst werden können.

Gemäß eines weiteren Ausführungsbeispiels ist vorgesehen, dass die mobile Rheometervorrichtung eine Temperiereinheit umfasst, sodass die mobile Rheometervorrichtung zumindest partiell benutzerdefiniert temperierbar ist.

Dies bietet den Vorteil, dass nachfolgende rheologische Messvorgänge, wenn für die jeweilige Methode vorteilhaft, optional vorbereitet werden können, in dem die mobile Rheometervorrichtung - und somit auch eine unmittelbare Umgebung dieser an ihrem Messort im Körper - temperiert werden kann. Dieser Effekt kann zudem auch für therapeutische Zwecke eingesetzt werden. Die Temperiereinheit kann dabei ausgelegt sein, Hitze, Kälte oder wahlweise beide dieser Optionen bereitzustellen.

Gemäß eines weiteren Ausführungsbeispiels ist vorgesehen, dass die mobile Rheometervorrichtung eine Temperaturmesseinheit umfasst, sodass eine jeweils aktuelle Temperatur zumindest eines Teilbereichs des inneren Bestandteils des menschlichen, tierischen oder pflanzlichen Körpers, in welchem sich die mobile Rheometervorrichtung befindet, messbar ist.

Somit kann die mobile Rheometervorrichtung mit Vorteil eingesetzt werden, um einen jeweiligen Temperaturverlauf in dem inneren Bestandteil des menschlichen, tierischen oder pflanzlichen Körper zu tracken, in welchem sie platziert und aktiviert wurde. Auf diese Weise können etwa inflammatorische Prozesse sehr genau verfolgt werden, wobei die erhobenen Messwerte zusätzlich zu den erhobenen rheologischen Messwerten beziehungsweise Parametern mit Vorteil für eine genauere Analyse beziehungsweise einer noch spezifischeren Diagnostik verwendet werden können.

Gemäß eines weiteren Ausführungsbeispiels ist vorgesehen, dass die rheologische Messeinrichtung mittels wenigstens einer Bewegungseinrichtung der mobilen Rheometervorrichtung ausrichtbar gelagert ist, sodass sie in Richtung eines zu bemessenden Bereichs im inneren Bestandteil des menschlichen, tierischen oder pflanzlichen Körpers auslenkbar vorgesehen ist.

Diese Ausführung kann insbesondere dann von Vorteil sein, wenn bei der Platzierung ein zunächst erreichter Messort als nicht optimal angesehen wird. Somit kann mittels der wenigstens einen Bewegungseinrichtung zügig und mit hohem Detailierungsgrad eine erneute Ausrichtung der Messeinrichtung erreicht werden, sodass die anschließend zu erhebenden Messwerte bestmöglich ermittelt beziehungsweise gemessen werden können.

Gemäß eines weiteren Ausführungsbeispiels ist vorgesehen, dass die computerbasierte Funktionseinheit mit Anwenderprogramm ausgelegt ist, die gemessenen rheologischen Parameter von dem menschlichen, tierischen oder pflanzlichen Körper hinsichtlich wenigstens einer medizinischen Diagnose zu klassifizieren, sodass einem Anwender der mobilen Rheometervorrichtung eine differenzierte Aussage des zu bemessenden Bereichs des menschlichen, tierischen oder pflanzlichen Körpers bereitstellbar ist.

Diese Ausführungsvariante der vorgestellten mobilen Rheometervorrichtung bietet den Vorteil, dass aufgrund der speziell vorgesehenen Auswerteeinheit eine Klassifizierung von Messwerten unmittelbar vor Ort durchführbar ist. Die mobile Rheometervorrichtung misst somit nicht nur innerhalb des jeweiligen Körpers, sondern liefert auch erste Ergebnisse, da die Klassifizierung eine erste Einschätzung im Hinblick auf wenigstens eine medizinische Diagnose liefert.

Gemäß eines weiteren Ausführungsbeispiels ist vorgesehen, dass die computerbasierte Funktionseinheit mit Anwenderprogramm mit wenigstens einer Datenbank für die medizinische Diagnostik von menschlichen, tierischen oder pflanzlichen Körpern basierend auf rheologischen Parametern verbindbar ist oder wenigstens eine solche Datenbank umfasst.

Auf diese Weise ist eine besonders schnelle und umfassende Klassifizierung durchführbar. Auch lässt sich je nach eingesetzter Datenbank das Einsatzgebiet der vorgestellten mobilen Rheometervorrichtung ausweiten. Zudem erhöht sich somit die Wahrscheinlichkeit bei einem Einsatz in entlegeneren Gebieten, dass die Verwendung der erfindungsgemäßen mobilen Rheometervorrichtung für eine folgerichtige Analytik als ausreichend einzustufen ist.

Dabei können etwa die rheologischen Eigenschaften mit der Datenbank (oder mit mehreren Datenbanken) mit Informationen zu den rheologischen Eigenschaften physiologischer oder pathologischer Natur abgeglichen werden. Durch den Abgleich erlaubt die Rheometervorrichtung die Beschreibung eines Hinweises oder mehrerer Hinweise für die Diagnostik (oder für den professionellen medizinischen Anwender) in Bezug auf den Zustand der Probe. Auch können Hinweise allgemeiner Natur in Form von Parametern oder dergleichen abgeleitet werden, die auch für einen medizinischen Laien leicht verständlich und zugänglich sind.

Mittels der vorgestellten mobilen Rheometervorrichtung und dem entsprechenden Verfahren ist es somit möglich, rheologische Untersuchungen mit Bezug zu einer medizinische Diagnostik von menschlichen, tierischen oder pflanzlichen Proben in unmittelbarer räumlicher und zeitlicher Nähe durchzuführen. Die rheologischen Untersuchungen erlauben dabei etwa Messungen unter möglichst physiologischen Bedingungen, zum Beispiel hinsichtlich eines pH-Wertes, einer lonenkonzentration, in Bezug auf vorhandene Enzyme oder weiteren Parametern etwa von Körperflüssigkeiten.

Gemäß eines weiteren Ausführungsbeispiels ist vorgesehen, dass die mobile Rheometervorrichtung ausgelegt ist, mittels Katheterisierungstechnologie in dem Inneren des menschlichen, tierischen oder pflanzlichen Körpers platziert zu werden.

Somit kann nicht nur eine sehr zielgenaue Platzierung erfolgen, da diese Technologie geeignet ist, punktgenau Verortungsvorgänge zu unterstützen, sondern es kann auch sichergestellt werden, dass die mobile Rheometervorrichtung während dieses Transports zu einem ersten Messort schonend und ohne die Gefahr von Transportschäden implementiert werden kann.

Als Katheterisierungstechnologie im weitesten Sinne der medizintechnischen Fachsprache kann jedwede Technologie verstanden werden, welche es erlaubt, eine medizintechnische Einheit, beispielsweise in Form der erfindungsgemäßen mobilen Rheometervorrichtung, mittels einer Röhrenvorrichtung, Schlauchvorrichtung oder dergleichen in das Innere eines Körpers zu transportieren.

In diesem Zusammenhang bedeutet dieser Fachbegriff nicht nur das eigentliche Legen dieser Röhrenvorrichtung, Schlauchvorrichtung oder dergleichen in den Körper, sondern auch den Transport der mobilen Rheometervorrichtung durch diesen Katheter hindurch sowie jegliche Hilfsmittel und Verfahrensschritte, um die mobile Rheometervorrichtung erstmalig in einem inneren Bestandteil des menschlichen, tierischen oder pflanzlichen Körpers zu platzieren.

Gemäß eines weiteren Ausführungsbeispiels ist vorgesehen, dass die mobile Rheometervorrichtung ausgelegt ist, durch orale Aufnahme in den menschlichen oder tierischen Körper platziert zu werden.

Dies kann etwa damit einhergehen, dass die vorgesehene mobile Rheometervorrichtung in dieser Ausführung ein gewisses Mindestaußenmaß nicht überschreitet, welche für einen Patienten (Mensch oder Tier) eine orale Aufnahme noch möglich erscheinen lässt. Der Vorteil besteht darin, dass eine Platzierung ohne weitere Techniken sofort und unmittelbar erreicht werden kann, ohne dass dem Patienten zusätzliche Aufwendungen zugemutet werden müssten.

Gemäß eines weiteren Ausführungsbeispiels ist vorgesehen, dass die Außenmaße der mobilen Rheometervorrichtung im Bereich zwischen 2 m x 2 m und 0,2 m x 0,2 m, vorzugsweise zwischen 1,5 m x 2 m und 0,25 m x 0,25 m, vorzugsweise zwischen 1 m x 1,5 m und 0,3 m x 0,3 m, vorzugsweise zwischen 1,5 m x 1,5 m und 0,3 m x 0,3 m, vorzugsweise zwischen 0,5 m x 0,5 m und 0,5 m x 0,5 m vorgesehen sind.

Vorteilhaft kann somit ein breites Einsatzspektrum der mobilen Rheometervorrichtung bereitgestellt werden. Auch der Transport zum Einsatzort kann somit besonders einfach geschehen, da die mobile Rheometervorrichtung auch für fragile Transportvorgänge keinen nennenswert großen Platzbedarf aufweist. Beispielsweise können auch Transporte via Drohnenlieferungstechnik oder dergleichen in Betracht gezogen werden. Ein sich einstellendes Gesamtgewicht der Vorrichtung kann etwa zwischen 45 kg und 250 kg, vorzugsweise zwischen 45 kg und 150 kg, vorzugsweise zwischen 45 kg und 100 kg, vorzugsweise zwischen 45 kg und 70 kg vorgesehen sein.

### KURZBESCHREIBUNG DER ZEICHNUNGEN

Im Folgenden wird die Erfindung unter Bezugnahme auf die Figuren der Zeichnungen erläutert. Von den Figuren zeigen:
- Fig. 1: eine schematische Darstellung einer mobilen Rheometervorrichtung nach einem Ausführungsbeispiel der vorliegenden Erfindung;
- Fig. 2: eine schematische Darstellung eines Anwenderszenarios einer mobilen Rheometervorrichtung nach einem Ausführungsbeispiel der vorliegenden Erfindung;
- Fig. 3: eine schematische Darstellung eines weiteren Anwenderszenarios einer mobilen Rheometervorrichtung nach einem Ausführungsbeispiel der vorliegenden Erfindung;
- Fig. 4: eine schematische Darstellung eines Systems zur Untersuchung von rheologischen Eigenschaften von menschlichen, tierischen oder pflanzlichen Bestandteilen;
- Fig. 5: ein schematisches Flussdiagramm für ein Verfahren nach einem Ausführungsbeispiel der vorliegenden Erfindung.

In den Figuren bezeichnen dieselben Bezugszeichen gleiche oder funktionsgleiche Komponenten, soweit nichts Gegenteiliges angegeben ist.

### AUSFÜHRLICHE BESCHREIBUNG VON AUSFÜHRUNGSBEISPIELEN DER ERFINDUNG

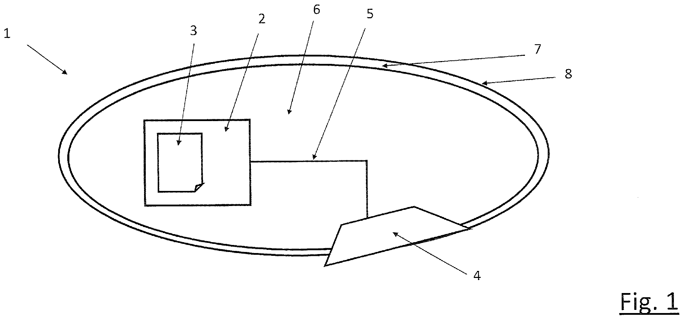

Fig. 1 zeigt eine schematische Darstellung einer mobilen Rheometervorrichtung 1 nach einem Ausführungsbeispiel der vorliegenden Erfindung. Diese mobile Rheometervorrichtung 1 kann zur Untersuchung von rheologischen Eigenschaften von menschlichen, tierischen oder pflanzlichen Bestandteilen verwendet beziehungsweise herangezogen werden.

Beispielsweise kann es sich dabei um jeweils innere Bestandteile eines menschlichen, tierischen oder pflanzlichen Körpers handeln. Eine äußere Anwendung beziehungsweise Verwendung ist damit nicht ausgeschlossen und kann im Zuge von Kalibrierungsvorgängen vor einem eigentlichen inneren Einsatz möglich sein.

Beispiele für innere Bestandteile von menschlichen oder tierischen Körpern können etwa Bestandteile von Organen oder sogar Organe selbst sein. So kann die mobile Rheometervorrichtung 1 etwa in einem Magen oder in Blutgefäßen eingesetzt werden. Beispielsweise kann die mobile Rheometervorrichtung 1 ausgelegt sein, um Untersuchungen von Bulkeigenschaften einer Magenschleimhaut durchzuführen.

Hierzu kann die mobile Rheometervorrichtung 1 etwa direkt in oder auf einem Gewebe des Magens, etwa der Magenschleimhaut, platziert werden. Ebenso kann eine Ausprägung der mobilen Rheometervorrichtung 1 vorgesehen sein, um grenzflächenrheologische Eigenschaften, wie beispielsweise an einer Phasengrenze einer wässrigen Phase und einer öligen Phase verwendet zu werden.

Ebenso kann eine Ausprägung der mobilen Rheometervorrichtung 1 vorgesehen sein, um etwa Messungen direkt in einem Flüssigkeitsvolumen, wie beispielsweise einer wässrigen Phase im Mageninhalt, vorzunehmen beziehungsweise für diese Zwecke verwendet zu werden.

Mit anderen Worten können mögliche Orte für die mobile Rheometervorrichtung 1 im Zusammenhang mit einem menschlichen oder tierischen Organismus etwa Blutgefäße oder Bestandteile eines Verdauungstrakts sein.

Auch Bestandteile einer Haut können mögliche Einsatzorte sein. Auch andere Organe oder Bestandteile des Körpers, wie beispielsweise die Blase oder dergleichen, können mögliche Einsatzorte der mobilen Rheometervorrichtung 1 sein.

Ziel der durchzuführenden rheologischen Messungen können dabei sowohl die Organe an sich als auch jegliche Körperflüssigkeiten oder Mischungen aus Körperflüssigkeiten und extern zugeführten Materialien, wie beispielsweise eine spezielle Nahrung oder allgemein jegliche Nahrungsbestandteile oder auch medizinische Bestandteile oder allgemein Medikamente oder Kombinationen aus dem zuvor genannten, sein.

Auch kann ein Einsatzort im Zusammenhang mit einem weiteren zu platzierenden Implantat vorgesehen sein.

Auch ist denkbar, dass die mobilen Rheometervorrichtung 1 im Inneren eines Knochen platziert wird, wobei hierfür beispielsweise erst eine Kavität oder dergleichen im Knochen vorgesehen wird und anschließend eine Platzierung mit oder ohne Hilfsmittel in dieser Kavität oder dergleichen im Knochen vorgenommen wird, um entweder eine temporäre oder sogar eine dauerhafte Platzierung der mobilen Rheometervorrichtung 1 vorzunehmen beziehungsweise durchzuführen.

Die mobile Rheometervorrichtung 1 ist in Fig. 1 mit einer eigenen computerbasierten Funktionseinheit 2 mit Anwenderprogramm 3 und einer rheologischen Messeinrichtung 4 dargestellt.

Die rheologische Messeinrichtung 4 ist dabei mit der computerbasierten Funktionseinheit 2 mit Anwenderprogramm 3 gekoppelt dargestellt, wobei für diese Zwecke eine erste Verbindungsleitung 5 zwischen diesen Komponenten vorgesehen ist.

In einer nicht näher dargestellten Ausführungsvariante ist vorstellbar, dass auch mehr als eine erste Verbindungsleitung 5 vorgesehen sind, um etwa eine stets sichere Verbindung zu gewährleisten, falls eine Hauptverbindung beschädigt oder unterbrochen sein sollte. Auch ist vorstellbar, dass dabei jegliche Schnittstelleneinrichtungen oder zumindest teilweise funkbasierte Verbindungstechnologien vorgesehen sind.

Die rheologische Messeinrichtung 4 und die computerbasierte Funktionseinheit 2 mit Anwenderprogramm 3 sind ferner in einer Transporteinheit 6 der mobilen Rheometervorrichtung 1 angeordnet dargestellt. Insbesondere sind diese beiden Komponenten hinsichtlich ihrer jeweils äußeren Ausmaße im Wesentlichen in dieser Transporteinheit 6 angeordnet vorgesehen. Lediglich ein Randbereich der rheologischen Messeinrichtung 4 ragt zum Teil über eine äußere Begrenzung dieser Transporteinheit 6 hinaus.

Es ist vorstellbar, dass die rheologischen Messeinrichtung 4 mittels wenigstens einer nicht näher dargestellten Bewegungseinrichtung der mobilen Rheometervorrichtung 1 in der Transporteinheit 6 ausrichtbar gelagert ist, sodass eine äußere Begrenzung der rheologischen Messeinrichtung 4 in einem ersten Betriebszustand sowohl innerhalb der Transporteinheit 6 als auch in einem beliebig weiteren Betriebszustand zum Teil außerhalb der Transporteinheit 6 angeordnet vorgesehen sein kann.

Diese Betriebszustände können auch während einer Messung variieren oder variiert werden und somit Teil eines im Anwenderprogramm 3 vorgehaltenen Messprotokolls sein.

Die Transporteinheit 6 ist dabei ausgelegt, zumindest temporär in einem nicht näher dargestellten inneren Bestandteil eines menschlichen, tierischen oder pflanzlichen Körpers platziert zu werden, sodass die rheologische Messeinrichtung 4 mit dem menschlichen, tierischen oder pflanzlichen Körper unter Kontaktaufnahme interagieren kann.

Die Kontaktaufnahme kann dabei jeweils so vorgesehen sein, sodass eine rheologische Messung sowohl von Bestandteilen, beispielsweise von Gewebebereichen oder dergleichen, als auch von Flüssigkeiten, beispielsweise von Blut oder Magensaft oder sonstigen Körperflüssigkeiten von menschlichen oder tierischen Organismen, durchgeführt werden kann.

Sinngemäß ist vorstellbar, dass auch Bestandteile von Pflanzen und jeglichen Flüssigkeiten der zu bemessenden Pflanze im Zuge der rheologischen Messungen untersucht werden kann.

Die eigentliche rheologische Messung erfolgt zum Beispiel, indem die rheologische Messeinrichtung 4 selbst eine Spannung oder eine Deformation auf etwa das Gewebe oder eine Körperflüssigkeit aufgibt.

Alternativ kann die rheologische Messeinrichtung 4 in einer nicht näher dargestellten Ausführungsvariante extrakorporal oder durch ein weiteres Gerät zu Schwingen oder zu einer anders gestalteten Bewegung angeregt werden. Dies kann auch mittels wenigstens einer nicht näher dargestellten Bewegungseinrichtung der mobilen Rheometervorrichtung 1 vollzogen werden. Somit können beispielsweise Gewebe untersucht werden und so pathologische Prozesse diagnostiziert und zeitlich getrackt werden.

Es können auch Prozesse, wie beispielsweise Verdauungsprozesse, untersucht werden. In diesem Fall liegt der Fokus nicht zwingend auf dem Organ, dem Gewebe oder einer Körperflüssigkeit, sondern eher auf der Mischung eines Speisebreis mit Körperflüssigkeit.

Auch die Wirkung von Medikamenten, zum Beispiel bei einer Verabreichung (beziehungsweise Gabe) während einer Operation stellt eine mögliche Verwendung der erfindungsgemäßen mobilen Rheometervorrichtung 1 dar.

Auch kann die mobile Rheometervorrichtung 1 verwendet werden, um einen Zustand von beispielsweise Implantaten zu tracken und so frühzeitig eine Erkenntnis zu erlangen, wann ein Implantat beispielsweise entnommen oder sogar erneuert werden muss.

Ein vorrangiger Verwendungszweck der erfindungsgemäßen mobilen Rheometervorrichtung 1 besteht jedoch allgemein in der Erfassung von rheologischen Daten beziehungsweise Messwerten.

Sollte es jedoch aus medizinischer Sicht therapeutisch vorteilhaft sein, so kann die erfindungsgemäße mobile Rheometervorrichtung 1 auch therapeutisch, zum Beispiel durch das gezielte Aufbringen von Bewegungen, wie etwa Oszillation, oder das Aufbringen einer Wärme/Kälte genutzt werden.

Für diese Zwecke kann die erfindungsgemäße mobile Rheometervorrichtung 1 in einer nicht näher dargestellten Ausführungsvariante etwa wenigstens eine Temperiereinheit umfassen.

Auch kann die beweglich gelagerte rheologische Messeinrichtung 4 ausgelegt sein, Bewegungsimpulse in den zu untersuchenden Organismus zu übertragen, sodass diese Bewegungsimpulse für therapeutische Zwecke eingesetzt werden können. Dabei können einzelne Bewegungsimpulse oder auch Bewegungsimpulsmuster oder dergleichen vorgesehen werden.

Die erfindungsgemäße mobile Rheometervorrichtung 1 ist in Fig. 1 zudem mit einer Antriebseinheit 7 in Form einer beweglichen Außenmembranstruktur 8 dargestellt.

Diese Antriebseinheit 7 in Form einer beweglichen Außenmembranstruktur 8 ist dabei im Wesentlichen rund um die Transporteinheit 6 herum vorgesehen. Insofern ist sie außerhalb der Transporteinheit 6 an dieser angeordnet vorgesehen.

In einer nicht näher dargestellten Ausführungsvariante ist vorstellbar, dass sie außerhalb an zumindest einem Teilbereich der Transporteinheit 6 angeordnet vorgesehen ist. In jeglichen Ausführungen ist sie jedoch ausgelegt, mittels pulsierender Bewegungen der jeweiligen beweglichen Außenmembranstruktur 8 eine definierte Bewegung der mobilen Rheometervorrichtung 1 zu bewirken.

Dabei kann die bewegliche Außenmembranstruktur 8 für diese Zwecke mit der computerbasierten Funktionseinheit 2 mit Anwenderprogramm 3 gekoppelt vorgesehen sein.

Mit anderen Worten ist somit die mobile Rheometervorrichtung 1 mittels der computerbasierten Funktionseinheit 2 mit Anwenderprogramm 3 mittelbar und mittels der Antriebseinheit 7 in Form der beweglichen Außenmembranstruktur 8 unmittelbar beweglich vorgesehen. Dabei kann vorgesehen sein, dass die mobile Rheometervorrichtung 1 sowohl in Flüssigkeiten als auch auf im Wesentlichen festen Untergrund beweglich vorgesehen sein kann.

In einer nicht näher dargestellten Ausführungsvariante kann die Antriebseinheit 7 auch andere Formen aufweisen. Dies kann etwa eine Art Propellereinrichtung sein, welche im Wesentlichen aus organischem Material basierend vorgesehen sein kann. Auch konventionelle mechanische Einheiten, welche überwiegend aus anorganischem Material aufgebaut sind, können alternativ vorgesehen sein. Dies können etwa elektrifizierte Antriebseinheiten aus im Wesentlichen anorganischen Materialien sein.

Die Antriebseinheit 7 in Form einer beweglichen Außenmembranstruktur 8 kann in einer nicht näher dargestellten Ausführungsvariante auch mit einer externen Anregungseinheit mit Anwenderprogramm gekoppelt vorgesehen sein, sodass diese Anregungseinheit mit Anwenderprogramm letztendlich die Impulse sendet, sodass die Antriebseinheit 7 in Form einer beweglichen Außenmembranstruktur 8 anschließend die mobile Rheometervorrichtung 1 etwa von einem ersten Messort zu wenigstens einem weiteren Messort bewegt. Eine benutzerdefinierte Navigation mit jeglichen dabei sinnvollen Hilfsmitteln kann dabei vorgesehen sein.

Die Transporteinheit 6 zusammen mit der Antriebseinheit 7 in jeglichen Varianten können zusammen genommen auch als Vehikel bezeichnet werden. Dabei kann vorgesehen sein, dass das Vehikel optional so ausgestaltet ist, sodass eine zielgerichtete Navigation im zu untersuchenden Körper möglich ist. Dazu ist das Vehikel, beispielsweise aufgrund der für diese Zwecke besonders ausgebildeten Antriebseinheit 7, optional so gestaltet, sodass gerichtete Bewegungen mit einer bestimmten Geschwindigkeit oder Beschleunigung oder Ruck oder höheren zeitlichen Ableitungen des Ortes in dem jeweiligen Koordinatensystem möglich sind.

In weiteren nicht näher dargestellten Ausführungsvarianten kann die mobile Rheometervorrichtung 1 zudem nicht näher dargestellte Befestigungsmittel umfassen, welche ausgelegt sind, eine temporäre oder dauerhafte Befestigung der mobilen Rheometervorrichtung 1 in dem inneren Bestandteil des menschlichen, tierischen oder pflanzlichen Körpers zu ermöglichen.

Diese nicht näher dargestellten Befestigungsmittel können dabei ausgewählt sein aus: anorganische Befestigungsmittel; organische Befestigungsmittel; anorganische Befestigungsmittel, welche ausgelegt sind, eine mechanische Verbindung zu ermöglichen; organische Befestigungsmittel, welche ausgelegt sind, eine mechanische Verbindung zu ermöglichen; anorganische Befestigungsmittel, welche ausgelegt sind, eine lösbare mechanische Verbindung zu ermöglichen; organische Befestigungsmittel, welche ausgelegt sind, eine lösbare mechanische Verbindung zu ermöglichen; anorganische Befestigungsmittel, welche ausgelegt sind, eine formschlüssige Verbindung mit zumindest einem Teilbereich des inneren Bestandteils zu ermöglichen; organische Befestigungsmittel, welche ausgelegt sind, eine formschlüssige Verbindung mit zumindest einem Teilbereich des inneren Bestandteils zu ermöglichen; anorganische Befestigungsmittel, welche ausgelegt sind, eine kraftschlüssige Verbindung mit zumindest einem Teilbereich des inneren Bestandteils zu ermöglichen; organische Befestigungsmittel, welche ausgelegt sind, eine kraftschlüssige Verbindung mit zumindest einem Teilbereich des inneren Bestandteils zu ermöglichen; anorganische Befestigungsmittel, welche ausgelegt sind, eine stoffschlüssige Verbindung mit zumindest einem Teilbereich des inneren Bestandteils zu ermöglichen; organische Befestigungsmittel, welche ausgelegt sind, eine stoffschlüssige Verbindung mit zumindest einem Teilbereich des inneren Bestandteils zu ermöglichen.

Beispielsweise können jegliche der oben genannten organischen Befestigungsmittel basierend auf biologischen Fixierungsvorgängen beruhen und für diese Zwecke ausgelegt sein.

Insofern ist vorstellbar, dass die organischen Befestigungsmittel ausgelegt sind, mittels biologischer Brücken hin zu einem Gewebe oder sonst einem biologischen Bestandteil des zu untersuchenden Körpers jeweilige Befestigungen einzugehen, sodass letztendlich die mobile Rheometervorrichtung 1 im Wesentlichen ortsfest beispielsweise an einer ersten Messstell im zu untersuchenden Körper fixierbar beziehungsweise befestigbar ist.

Hierbei kann etwa ausgenutzt werden, dass menschliche Zellen eine bestimmte räumliche Charakteristik aufweisen, welche etwa dreidimensional in ein räumliches Volumen hervorragen, sodass dann mittels beispielsweise spezieller Proteinstrukturen der organischen Befestigungsmittel hier eine Bindung möglich ist.

Dies kann sinnbildlich etwa im Sinne eines Prinzips zweier Puzzleteile vorgesehen sein. Auch können allgemein solche organischen Verbindungen mittels entsprechender Molekülbrücken vorgesehen sein, wobei etwa ein Schlüssel-Schloss-Prinzip oder dergleichen vorgesehen sein kann. Dabei können unterschiedliche Ladungen an den zu verbindenden Partnern vorgesehen werden, sodass wenigstens eine entsprechende Molekülbrücke auf Basis von Ladeunterschieden vorgesehen sein kann.

Die erfindungsgemäße mobile Rheometervorrichtung 1 kann etwa durch Katheterisierung oder dergleichen oder durch orale Aufnahme oder durch manuelles Aufbringen im oder auf dem zu untersuchenden Organ in oder auf einem menschlichen, tierischen oder pflanzlichen Körper platziert werden. Auch ein operatives Platzieren der erfindungsgemäßen mobilen Rheometervorrichtung 1 ist vorstellbar. Dies kann jeweils mittels eines minimalinvasiven Verfahrens vorgesehen sein.

Beispielsweise in dem ein Loch in einen Knochen gebohrt wird, anschließend die mobile Rheometervorrichtung 1 in das Loch platziert wird und zum Schluss eine Art organischer Zement oder ein verträglicher Klebstoff oder ein sonstiges Hilfsmittel zum Befestigen der mobilen Rheometervorrichtung 1 in dem Loch verwendet wird.

Für die Auswertung kann optional auf ein Datenbanksystem zugegriffen werden, wobei solch ein Datenbanksystem beispielsweise global oder lokal auf einem (mobilen) Endgerät, einer Cloudeinrichtung oder auch einem Praxis (-Verbund) oder Klinik-Netzwerk verfügbar sein kann.

Auf Grundlage dieses Datenbanksystems kann eine Diagnostik oder das Beschreiben von Trends von physiologischen oder pathologischen Zuständen möglich sein, die im Zusammenhang mit bestimmten (Verdachts-)Diagnosen stehen.

Optional kann eine Datenübertragung auf ein mobiles Endgerät erfolgen, zum Beispiel wenn ein Patient als Laie einen eigenen Krankheitsverlauf trackt.

Zur Auswertung können gemessene Daten beziehungsweise Messwerte entweder in einer nicht näher dargestellten Speichereinheit der mobilen Rheometervorrichtung 1 gespeichert werden (zum Beispiel über die Dauer einer bestimmten Zeit) und anschließend ausgewertet werden.

Alternativ kann vorgesehen sein, dass eine Datenübertragung mittels eines nicht näher dargestellten Sende- und Empfangsmoduls der mobilen Rheometervorrichtung 1 in Form einer Datenübertragung erfolgt. Dies kann bereits während der Zeit geschehen, in welcher die mobile Rheometervorrichtung 1 sich noch in dem zu bemessenden Körper befindet.

Insofern ist auch eine Datenübertragung in Echtzeit mit entsprechender Darstellung auf einer Ansichtsvorrichtung, einer Monitoreinheit oder dergleichen vorstellbar.

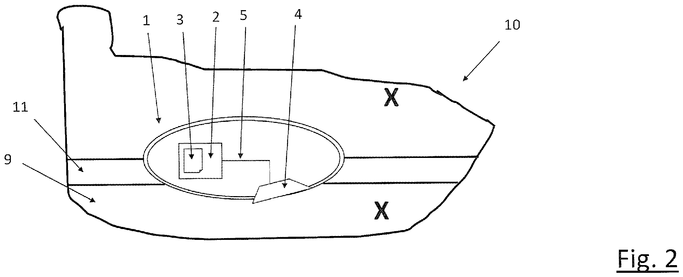

Fig. 2 zeigt eine schematische Darstellung eines Anwenderszenarios einer mobilen Rheometervorrichtung 1 nach einem Ausführungsbeispiel der vorliegenden Erfindung. Es kann sich dabei etwa um jeweilige Ausführungsvarianten handeln, welche in der Beschreibung zu Figur 1 erwähnt worden sind.

Gleiche Bezugszeichen bezeichnen insofern gleiche Merkmale, sodass diese hier nicht erneut eingeführt werden.

Die in Fig. 2 dargestellte mobile Rheometervorrichtung 1 schwimmt dabei in einer wässrigen Phase 9 eines menschlichen Magens 10 auf, wobei sie zum Teil auch in einer auf der wässrigen Phase 9 liegenden öligen Phase 11 aufschwimmt.

Die beiden X-Symbole verdeutlichen mögliche weitere Orte, an denen die mobile Rheometervorrichtung 1 mittels nicht näher dargestellten Befestigungsmittel innerhalb des Magens 10 fixiert werden kann.

Bezogen auf die Bildebene verdeutlich das untenstehende X-Symbol, dass die mobile Rheometervorrichtung 1 auch gänzlich in der wässrigen Phase 9 des Magens 10 angeordnet sein kann. Entsprechend verdeutlicht das obere X-Symbol, wiederum bezogen auf die Bildebene, dass die mobile Rheometervorrichtung 1 auch gänzlich außerhalb sowohl der wässrigen Phase 9 als auch der öligen Phase 11 angeordnet sein kann.

Dort kann sie etwa vorgesehen sein, um Bulkeigenschaften eines Bereichs der Magenschleimhaut des Magens 10 zu untersuchen. Entsprechend können in der wässrigen Phase 9 Bulkeigenschaften in diesem Bereich der Magenschleimhaut des Magens 10 untersucht werden.

Es ist vorstellbar, dass in nicht näher gezeigten Ausführungsbeispielen die mobile Rheometervorrichtung 1 solche Standortverlagerungen mittels der Antriebseinheit 7 in Form einer beweglichen Außenmembranstruktur 8 bewältigt, wobei lösbare Befestigungsmittel von Vorteil sind, sodass im Anschluss an eine Standortverlagerung wiederum eine ortsfeste Fixierung möglich ist, ohne dass dabei Strukturen der mobilen Rheometervorrichtung 1 beschädigt werden könnten.

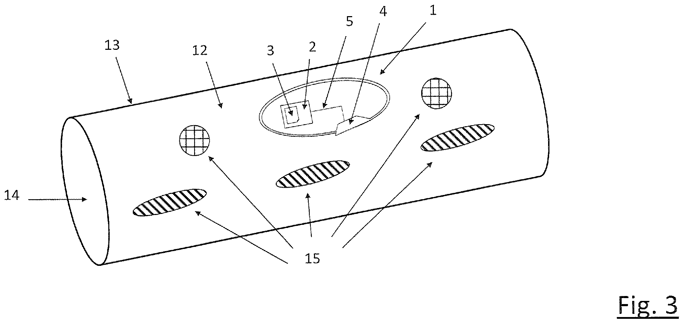

Fig. 3 zeigt eine schematische Darstellung eines weiteren Anwenderszenarios einer mobilen Rheometervorrichtung 1 nach einem Ausführungsbeispiel der vorliegenden Erfindung.

Es kann sich dabei etwa um jeweilige Ausführungsvarianten handeln, welche in der Beschreibung zu Figur 1 erwähnt worden sind. Gleiche Bezugszeichen bezeichnen insofern gleiche Merkmale, sodass diese hier nicht erneut eingeführt werden.

In der Fig. 3 ist die mobile Rheometervorrichtung 1 besonders klein vorgesehen, wobei sie beispielsweise eine maximale äußere Ausdehnung von bis maximal einer Hälfte eines Durchmessers eines größeren Blutgefäßes aufweisen kann. Insofern ist eine maximale äußere Ausdehnung von bis zu 500 Mikrometer vorstellbar.

In Fig. 3 ist die mobile Rheometervorrichtung 1 schematisch an einer inneren Wandung 12 eines Blutgefäßes 13, beispielsweise einer großen menschlichen Arterie, angeordnet dargestellt.

Dies kann beispielsweise mittels nicht näher dargestellter anorganischer oder organischer Befestigungsmittel vorgesehen sein.

Eine nicht näher dargestellte beziehungsweise nicht einsehbare Sensorfläche der rheologischen Messeinrichtung 4 ist dabei in Richtung eines Innenraums 14 des Blutgefäßes 13 zugewandt, sodass rheologische Messung an der vorbeiströmenden Flüssigkeit, sprich dem menschlichen Blut mit seinen Bestandteilen (schematisch stark vereinfacht und nicht maßstabsgetreu dargestellt mittels dessen Blutbestandteile 15), möglich sind.

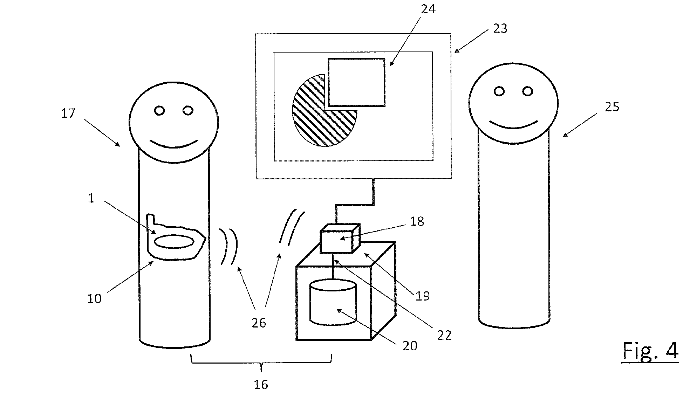

Fig. 4 zeigt eine schematische Darstellung eines Systems 16 zur Untersuchung von rheologischen Eigenschaften von menschlichen, tierischen oder pflanzlichen Bestandteilen.

Eine mobile Rheometervorrichtung 1 nach einem Ausführungsbeispiel der vorliegenden Erfindung ist dabei in einem Magen 10 eines Patienten 17 dargestellt.

In einer nicht näher dargestellten Ausführungsvariante könnten in dem Patienten 17 mehr als eine mobile Rheometervorrichtung 1 innerhalb seines Körpers vorgesehen sein.

Das dargestellte System 16 ist dabei zur Untersuchung von rheologischen Eigenschaften von menschlichen, tierischen oder pflanzlichen Bestandteilen ausgelegt und umfasst für diese Zwecke neben der erfindungsgemäßen mobilen Rheometervorrichtung 1 eine mit der zumindest einen mobilen Rheometervorrichtung 1 kontaktlos koppelbaren externen Steuereinrichtung 18 mit Applikationsprogramm, welche auf einer Ablagestruktur 19 neben dem Patienten 17 dargestellt ist.

Die Ablagestruktur 19 kann etwa ein Tisch oder ein jegliches Möbelstück sein, wie es in einem Krankenhaus, insbesondere in einem Operationssaal eines Krankenhauses, Verwendung findet.

Unterhalb (bezogen auf die Bildebene) der externen Steuereinrichtung 18 ist in der Ablagestruktur 19 eine Datenbank 20 stark vereinfacht und lediglich symbolisch dargestellt.

Diese Datenbank 20 ist mit der externen Steuereinrichtung 18 über eine zweite Verbindungsleitung 21 gekoppelt dargestellt. Bei der Datenbank 20 handelt es sich um eine computertechnische Einrichtung für die medizinische Diagnostik von Bestandteilen eines menschlichen, tierischen oder pflanzlichen Körpers basierend auf rheologischen Parametern.

In einer nicht näher dargestellten Variante ist vorstellbar, dass mehr als eine Datenbank 20 mit der externen Steuereinrichtung 18 gekoppelt vorgesehen ist. Beispielsweise kann es sich dabei um verschiedene Datenbanken aus einem Praxisverbund, einer Cloudeinrichtung, eines Krankenhausnetzwerkes, eines Forschungsnetzwerkes und dergleichen handeln.

Über eine dritte Verbindungsleitung 22 ist die externe Steuereinrichtung 18 mit einer Anzeigeeinheit 23 gekoppelt dargestellt. Auf der Anzeigeeinheit 23, die optional in einer Ausführungsvariante aktiver Bestandteil des Systems 16 ist, wird stark vereinfacht ein Diagnosebild 24 der rheologischen Untersuchung dargestellt.

Es ist auch vorstellbar, dass die Anzeigeeinheit 23 als separate Komponente vorgesehen ist und das System 16 lediglich über eine externe Steuereinrichtung 18 verfügt, welche mit den üblichen Schnittstelleneinrichtungen zur Kopplung von weiteren externen Geräten ausgestattet ist.

Die Anzeigeeinheit 23 kann in jedem der oben genannten Fälle beispielsweise eine Art Flachbildschirmeinheit oder dergleichen sein. Eine medizinische Fachkraft 25 steht (bezogen auf die Bildebene) rechts von der Anzeigeeinheit 23 und begutachtet das aktuell angezeigte Diagnosebild 24.

Dargestellte Funkwellensymbole 26 verdeutlichen, inwiefern die mobile Rheometervorrichtung 1 innerhalb des Körpers des Patienten 17 mit der externen Steuereinrichtung 18 kommuniziert.

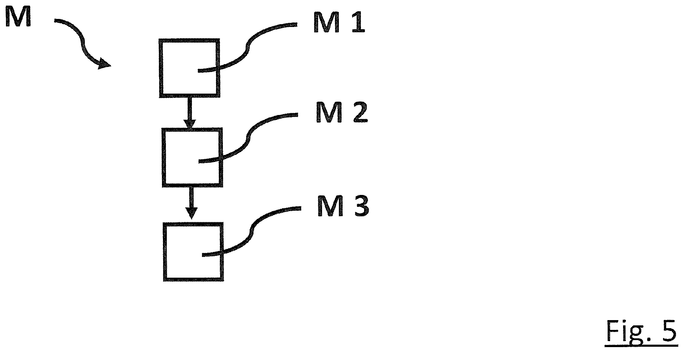

Fig. 5 zeigt ein schematisches Flussdiagramm für ein Verfahren M zur Untersuchung von rheologischen Eigenschaften von menschlichen, tierischen oder pflanzlichen Bestandteilen. In einem ersten Verfahrensschritt M1 wird eine erfindungsgemäße mobile Rheometervorrichtung 1 bereitgestellt und aktiviert. In einem zweiten Verfahrensschritt M2 wird die erfindungsgemäße mobile Rheometervorrichtung 1 in oder an einem Bestandteil von einem menschlichen, tierischen oder pflanzlichen Körper platziert. In einem dritten Verfahrensschritt M3 wird wenigstens eine Funktion der rheologischen Messeinrichtung 4 von der mobilen Rheometervorrichtung 1 zur Interaktion mit wenigstens einem Teilbereich des Bestandteils des menschlichen, tierischen oder pflanzlichen Körpers ausgelöst, sodass wenigstens ein rheologischer Parameter mittels der rheologischen Messeinrichtung gemessen und bereitgestellt wird.

### BEZUGSZEICHENLISTE

- 1: mobile Rheometervorrichtung
- 2: computerbasierte Funktionseinheit
- 3: Anwenderprogramm
- 4: rheologische Messeinrichtung
- 5: erste Verbindungsleitung
- 6: Transporteinheit
- 7: Antriebseinheit
- 8: Außenmembranstruktur
- 9: wässrige Phase
- 10: Magen
- 11: ölige Phase 11
- 12: innere Wandung
- 13: Blutgefäß
- 14: Innenraum
- 15: Blutbestandteil
- 16: System
- 17: Patient
- 18: externe Steuereinrichtung
- 19: Ablagestruktur
- 20: Datenbank
- 21: zweite Verbindungsleitung
- 22: dritte Verbindungsleitung
- 23: Anzeigeeinheit
- 24: Diagnosebild
- 25: medizinische Fachkraft
- 26: Funkwellensymbol
- M: Verfahren
- M1: Verfahrensschritt
- M2: Verfahrensschritt
- M3: Verfahrensschritt

## Patentansprüche

1. Mobile Rheometervorrichtung (1) zur Untersuchung von rheologischen Eigenschaften von menschlichen, tierischen oder pflanzlichen Bestandteilen umfassend eine rheologische Messeinrichtung (4) und eine damit gekoppelte computerbasierte Funktionseinheit (2) mit Anwenderprogramm (3) **dadurch gekennzeichnet, dass** die mobile Rheometervorrichtung (1) eine Transporteinheit (6) umfasst, in welcher die rheologische Messeinrichtung (4) und die damit gekoppelte computerbasierte Funktionseinheit (2) angeordnet sind, wobei die Transporteinheit (6) ausgelegt ist, zumindest temporär in einem inneren Bestandteil eines menschlichen, tierischen oder pflanzlichen Körpers platziert zu werden, sodass die rheologische Messeinrichtung (4) mit dem menschlichen, tierischen oder pflanzlichen Körper unter Kontaktaufnahme interagieren kann.

2. Mobile Rheometervorrichtung (1) nach Anspruch 1, wobei die Transporteinheit (6) Befestigungsmittel umfasst, welche ausgelegt sind, eine temporäre oder dauerhafte Befestigung der mobilen Rheometervorrichtung (1) in dem inneren Bestandteil des menschlichen, tierischen oder pflanzlichen Körpers zu ermöglichen.

3. Mobile Rheometervorrichtung (1) nach Anspruch 2, wobei die Befestigungsmittel ausgewählt sind aus: anorganische Befestigungsmittel; organische Befestigungsmittel; anorganische Befestigungsmittel, welche ausgelegt sind, eine mechanische Verbindung zu ermöglichen; organische Befestigungsmittel, welche ausgelegt sind, eine mechanische Verbindung zu ermöglichen; anorganische Befestigungsmittel, welche ausgelegt sind, eine lösbare mechanische Verbindung zu ermöglichen; organische Befestigungsmittel, welche ausgelegt sind, eine lösbare mechanische Verbindung zu ermöglichen; anorganische Befestigungsmittel, welche ausgelegt sind, eine formschlüssige Verbindung mit zumindest einem Teilbereich des inneren Bestandteils zu ermöglichen; organische Befestigungsmittel, welche ausgelegt sind, eine formschlüssige Verbindung mit zumindest einem Teilbereich des inneren Bestandteils zu ermöglichen; anorganische Befestigungsmittel, welche ausgelegt sind, eine kraftschlüssige Verbindung mit zumindest einem Teilbereich des inneren Bestandteils zu ermöglichen; organische Befestigungsmittel, welche ausgelegt sind, eine kraftschlüssige Verbindung mit zumindest einem Teilbereich des inneren Bestandteils zu ermöglichen; anorganische Befestigungsmittel, welche ausgelegt sind, eine stoffschlüssige Verbindung mit zumindest einem Teilbereich des inneren Bestandteils zu ermöglichen; organische Befestigungsmittel, welche ausgelegt sind, eine stoffschlüssige Verbindung mit zumindest einem Teilbereich des inneren Bestandteils zu ermöglichen.

4. Mobile Rheometervorrichtung (1) nach Anspruch 2 oder 3, wobei die Befestigungsmittel zumindest teilweise auf einer Oberfläche der Transporteinheit (6) angeordnet sind.

5. Mobile Rheometervorrichtung (1) nach einem der Ansprüche 2 bis 4, wobei die Befestigungsmittel organische Befestigungsmittel umfassen, welche ausgelegt sind, mittels wenigstens einem benutzerdefiniert eingebrachten Einfluss manipulierbar zu sein, sodass die organischen Befestigungsmittel an eine molekulare Struktur des inneren Bestandteils eines menschlichen, tierischen oder pflanzlichen Körpers anpassbar sind.

6. Mobile Rheometervorrichtung (1) nach Anspruch 5, wobei der benutzerdefiniert eingebrachte Einfluss ausgewählt ist aus: thermischer Einfluss, elektromechanischer Einfluss, mechanischer Einfluss, chemischer Einfluss, chemische, biologische oder physikalische Übertragung von Energie.

7. Mobile Rheometervorrichtung (1) nach einem der vorhergehenden Ansprüche, wobei die mobile Rheometervorrichtung (1) zumindest eine Antriebseinheit (7) umfasst, sodass die mobile Rheometervorrichtung (1) innerhalb des inneren Bestandteils des menschlichen, tierischen oder pflanzlichen Körpers bewegbar ausgestaltet ist.

8. Mobile Rheometervorrichtung (1) nach Anspruch 7, wobei die zumindest eine Antriebseinheit (7) ausgelegt ist, eine Bewegung der mobilen Rheometervorrichtung (1) in einer Flüssigkeit, in welcher sich die mobile Rheometervorrichtung (1) befindet, zu bewirken oder wobei die zumindest eine Antriebseinheit (7) ausgelegt ist, eine Bewegung der mobilen Rheometervorrichtung (1) auf einem im Wesentlichen festen Untergrund, auf welchem sich die mobile Rheometervorrichtung (1) befindet, zu bewirken oder wobei die zumindest eine Antriebseinheit (7) ausgelegt ist, eine Bewegung der mobilen Rheometervorrichtung (1) in einer Flüssigkeit, in welcher sich die mobile Rheometervorrichtung (1) befindet, und eine Bewegung der mobilen Rheometervorrichtung (1) auf einem im Wesentlichen festen Untergrund, auf welchem sich die mobile Rheometervorrichtung (1) befindet, zu bewirken.

9. Mobile Rheometervorrichtung (1) nach einem der vorhergehenden Ansprüche 7 oder 8, wobei die zumindest eine Antriebseinheit (7) zumindest eine Taptic-Motoreinheit umfasst.

10. Mobile Rheometervorrichtung (1) nach einem der vorhergehenden Ansprüche 7 bis 9, wobei die zumindest eine Antriebseinheit (7) ausgelegt ist, mittels der computerbasierten Funktionseinheit (2) gesteuert zu werden.

11. Mobile Rheometervorrichtung (1) nach einem der vorhergehenden Ansprüche 7 bis 10, wobei die zumindest eine Antriebseinheit (7) zumindest anteilig aus organischem Material besteht.

12. Mobile Rheometervorrichtung (1) nach einem der vorhergehenden Ansprüche 7 bis 11, wobei die zumindest eine Antriebseinheit (7) zumindest eine bewegliche Außenmembranstruktur (8) umfasst, welche außerhalb an zumindest einem Teilbereich der Transporteinheit (6) angeordnet ist, sodass mittels pulsierender Bewegungen der jeweiligen beweglichen Außenmembranstruktur (8) eine definierte Bewegung der mobilen Rheometervorrichtung (1) bewirkbar ist.

13. Mobile Rheometervorrichtung (1) nach Anspruch 12, wobei die zumindest eine bewegliche Außenmembranstruktur (8) mittels der für diese Zwecke mit der jeweiligen Außenmembranstruktur (8) gekoppelten computerbasierten Funktionseinheit (2) mit Anwenderprogramm (3) bewegbar ist oder wobei die zumindest eine bewegliche Außenmembranstruktur (8) mittels einer für diese Zwecke mit der jeweiligen Außenmembranstruktur (8) kontaktlos gekoppelten externen Anregungseinheit mit Anwenderprogramm bewegbar ist.

14. Mobile Rheometervorrichtung (1) nach einem der vorhergehenden Ansprüche 7 bis 13, wobei die zumindest eine Antriebseinheit (7) ausgelegt ist, eine benutzerdefinierte Geschwindigkeit, insbesondere eine benutzerdefiniert gleichmäßige Beschleunigung oder eine benutzerdefiniert negative Beschleunigung oder eine benutzerdefiniert positive Beschleunigung zu bewirken.

15. Mobile Rheometervorrichtung (1) nach einem der vorhergehenden Ansprüche 7 bis 14, wobei die zumindest eine Antriebseinheit (7) ausgelegt ist, eine jegliche Bewegung oder einen jeglichen Bewegungsablauf der mobilen Rheometervorrichtung (1) in einem dreidimensionalen Koordinatensystem zu bewirken.

16. Mobile Rheometervorrichtung (1) nach einem der vorhergehenden Ansprüche, wobei die computerbasierte Funktionseinheit (2) eine Speichereinheit zur Speicherung von rheologischen Messdaten umfasst.

17. Mobile Rheometervorrichtung (1) nach einem der vorhergehenden Ansprüche, wobei die computerbasierte Funktionseinheit (2) eine Sende- und Empfangsmodul umfasst, sodass die mobile Rheometervorrichtung (1) ausgelegt ist, erhobene rheologische Messdaten an zumindest ein externes Gerät zu übermitteln und ferner ausgelegt ist, mittels zumindest eines externen Geräts bedient zu werden.

18. Mobile Rheometervorrichtung (1) nach einem der vorhergehenden Ansprüche, wobei die mobile Rheometervorrichtung (1) eine Temperiereinheit umfasst, sodass die mobile Rheometervorrichtung (1) zumindest partiell benutzerdefiniert temperierbar ist.

19. Mobile Rheometervorrichtung (1) nach einem der vorhergehenden Ansprüche, wobei die mobile Rheometervorrichtung (1) eine Temperaturmesseinheit umfasst, sodass eine jeweils aktuelle Temperatur zumindest eines Teilbereichs des inneren Bestandteils des menschlichen, tierischen oder pflanzlichen Körpers, in welchem sich die mobile Rheometervorrichtung (1) befindet, messbar ist.

20. Mobile Rheometervorrichtung (1) nach einem der vorhergehenden Ansprüche, wobei die rheologische Messeinrichtung (4) mittels wenigstens einer Bewegungseinrichtung der mobilen Rheometervorrichtung (1) ausrichtbar gelagert ist, sodass sie in Richtung eines zu bemessenden Bereichs im inneren Bestandteil des menschlichen, tierischen oder pflanzlichen Körpers auslenkbar vorgesehen ist.

21. Mobile Rheometervorrichtung (1) nach einem der vorhergehenden Ansprüche, wobei die computerbasierte Funktionseinheit (2) mit Anwenderprogramm (3) ausgelegt ist, die gemessenen rheologischen Parameter von dem menschlichen, tierischen oder pflanzlichen Körper hinsichtlich wenigstens einer medizinischen Diagnose zu klassifizieren, sodass einem Anwender der mobilen Rheometervorrichtung (1) eine differenzierte Aussage des zu bemessenden Bereichs des menschlichen, tierischen oder pflanzlichen Körpers bereitstellbar ist.

22. Mobile Rheometervorrichtung (1) nach Anspruch 21, wobei die computerbasierte Funktionseinheit (2) mit Anwenderprogramm (3) mit wenigstens einer Datenbank (20) für die medizinische Diagnostik von menschlichen, tierischen oder pflanzlichen Körpern basierend auf rheologischen Parametern verbindbar ist oder wenigstens eine solche Datenbank (20) umfasst.

23. Mobile Rheometervorrichtung (1) nach einem der vorhergehenden Ansprüche, wobei die mobile Rheometervorrichtung (1) ausgelegt ist, mittels Katheterisierungstechnologie in dem Inneren des menschlichen, tierischen oder pflanzlichen Körpers platziert zu werden.

24. Mobile Rheometervorrichtung (1) nach einem der vorhergehenden Ansprüche 1 bis 22, wobei die mobile Rheometervorrichtung (1) ausgelegt ist, durch orale Aufnahme in den menschlichen oder tierischen Körper platziert zu werden.

25. Mobile Rheometervorrichtung (1) nach einem der vorhergehenden Ansprüche, wobei die Außenmaße der mobilen Rheometervorrichtung (1) im Bereich zwischen 2 m x 2 m und 0,2 m x 0,2 m, vorzugsweise zwischen 1,5 m x 2 m und 0,25 m x 0,25 m, vorzugsweise zwischen 1 m x 1,5 m und 0,3 m x 0,3 m, vorzugsweise zwischen 1,5 m x 1,5 m und 0,3 m x 0,3 m, vorzugsweise zwischen 0,5 m x 0,5 m und 0,5 m x 0,5 m vorgesehen sind.

26. System (16) zur Untersuchung von rheologischen Eigenschaften von menschlichen, tierischen oder pflanzlichen Bestandteilen umfassend zumindest eine mobile Rheometervorrichtung (1) gemäß einem der Ansprüche 1 bis 25, eine mit der zumindest einen mobilen Rheometervorrichtung (1) kontaktlos koppelbaren externen Steuereinrichtung (18) mit Applikationsprogramm und wenigstens einer mit der Steuereinrichtung (18) koppelbaren Datenbank (20) für die medizinische Diagnostik von Bestandteilen eines menschlichen, tierischen oder pflanzlichen Körpers basierend auf rheologischen Parametern.

27. Verfahren (M) zur Untersuchung von rheologischen Eigenschaften von menschlichen, tierischen oder pflanzlichen Bestandteilen umfassend:
• Bereitstellen und Aktivieren einer mobilen Rheometervorrichtung (1) nach einem der Ansprüche 1 bis 25;
• Platzieren der mobilen Rheometervorrichtung (1) in oder an einem Bestandteil von einem menschlichen, tierischen oder pflanzlichen Körper;
• Auslösen von wenigstens einer Funktion der rheologischen Messeinrichtung (4) von der mobilen Rheometervorrichtung (1) zur Interaktion mit wenigstens einem Teilbereich des Bestandteils des menschlichen, tierischen oder pflanzlichen Körpers, sodass wenigstens ein rheologischer Parameter mittels der rheologischen Messeinrichtung (4) gemessen und bereitgestellt wird.

28. Verwendung einer mobilen Rheometervorrichtung (1) nach einem der Ansprüche 1 bis 25 für Durchführungen von therapeutischen oder I<urierenden Maßnahmen in einem menschlichen, tierischen oder pflanzlichen Organismus.
